# EUROPEAN PATENT APPLICATION

(11) **EP 2 548 516 A1**
(43) Date of publication of application: **23.01.2013**
(21) Application number: 11174662.4
(22) Date of filing: 20.07.2011
(51) Int. Cl.: A61B 10/02, A61B 5/00, G01N 21/17, G01N 21/39, G02B 6/00

(54) **A system for enabling generation of photoacoustic images**

(71) Applicant: Universiteit Twente, 7522 NB Enschede (NL)
(72) Inventor: Manohar, Srirang, 7481 LN Haaksbergen (NL)
(74) Representative: Jansen, Cornelis Marinus

(57) **Abstract**

The invention relates to a system (10) for enabling generation of photoacoustic images, comprising a light source (3) arranged to generate pulsed or modulated light having a wavelength in the range of 200 - 2500 nm, a biopsy needle having a needle body (4) with a distal portion (4a), the needle body being provided with an optical fiber (5) having a distal portion (5a) and adapted to conduct the light from the light source (3) via the distal portion (5a) towards a target region (2) located inside a patient (1). Photoacoustic signals (7) generated in the target region (2) are advantageously intercepted by a suitable detector, such as a per se known ultrasonic probe (6). The ultrasonic probe (6) may be connected to a data processing system (8) for visualizing suitable images (9a) on a display (9). The invention further relates to a method of generating photoacoustic images and to a biopsy needle.

## Description

### BACKGROUND OF THE INVENTION

Minimally invasive pre-operative assessment of lesions is important in making diagnosis and in planning therapeutic strategies. The established method for assessing the lesions in various parts of the body is image guided core needle biopsy (CNB). In this method small samples of an abnormality are removed through a percutaneously inserted hollow needle which is guided to the lesion using sonography, X-ray stereotactic imaging or magnetic resonance imaging. In the case of breast tumors, 4 to 6 cores per lesion are removed with repeated insertions to obtain a sufficient sampling of the tumor.

The cores are examined histologically to ascertain factors such as tumor grade and tumor type which aid in diagnosis. Other parameters such as tumor size and vascular channel invasion may also be assessed, which in combination with tumor grade and type provide information regarding the prognosis for the patient. Additionally, the presence of molecular markers such as hormone receptors, growth factors receptors, tumor suppressor genes, oncogenes etc. can also be ascertained using appropriate histological stains. Specific marker expression can in certain situations provide information regarding patient-response to treatment.

Accordingly, it is of great importance that the samples are removed indeed from the target area, and not from the adjacent tissues. One of the methods to determine the type of a hidden tissue is the photoacoustic method. An embodiment of a system for generation of photoacoustic images is known from US2009/0156932. In the known system a pulsed laser is used for generating photoacoustic images, which is adapted to operate with the wavelength of 200 - 2500 nm. The known laser is coupled to an optical system comprising fibers and optical elements adapted to conduct the laser light towards suitable target regions. The known system is arranged to enable detection of target objects circulating in vessels.

It is a disadvantage of the known system that it may have reduced sensitivity and reliability for deep seated target objects.

An embodiment of a biopsy needle is known from WO 2009/150563. The known biopsy needle comprises an ultrasound transducer adapted to generate a narrow beam directed towards a tissue under investigation. The ultrasonic beam is generated by the transducer and is used for improving localization of the biopsy needle for extracting suitable biological material. The known biopsy needle may further comprise an optical fiber for enabling suitable spectroscopic analysis of the reflected ultrasound radiation. It is a disadvantage of the known needle that it is relatively complex, which increases manufacturing costs.

### SUMMARY OF THE INVENTION

It is an object of the invention to provide a system for enabling generation of photoacoustic images, which is relatively simple yet reliable. It is a still further object of the invention to provide a system for generation of photoacoustic images having an increased sensitivity to various tissue types. Still alternatively, it is an object of the invention to provide an improved biopsy needle for enabling percutaneous tissue examination.

To this end the system for enabling generation of photoacoustic images comprises a light source, a biopsy needle having a needle body with a distal portion, the needle body being provided with an optical fiber adapted to conduct light from the light source via the distal portion towards a target region preferably in the proximity of the target, wherein the light source comprises a pulsed or modulated light source having a wavelength in the range of 200 ― 2500 nm.

It is found that by adapting a biopsy needle to enable a deep seated photoacoustic investigation a more accurate tissue mapping and identification may be achieved. It is further found that with the system of the invention tumor sizing is possible as well as accurate tumor margin demarcation, which may increase the success rate of a suitable treatment, such as surgery or radiotherapy. It will be appreciated that the needle may be insertable percutaneously.

On of the hallmarks of cancer is the presence of enhanced vascularisation due to the process of angiogenesis to support the high metabolism of the tumor cells. Wavelengths of light in the range of 200 - 2500 nm are particularly suited to excite photoacoustic signals by predominantly haemoglobin and oxy-haemoglobin adsorption which give malignancies an intrinsic contrast to light. Wavelengths of light could also be selected to excite photoacoustic signals by water absorption or by fat absorption or combinations thereof. Photoacoustic image or images will then represent a mapping of the tissue chromphore presence, which is different depending on the dignity of the abnormality (malignant or otherwise) compared with healthy tissue. With the use of estimation methods in multi-wavelength approaches, concentrations of tissue chromophores can be calculated. Further functional information such as concentration of oxygen associated with haemoglobin can also be estimated. Photoacoustic images will then represent a mapping of these concentrations. Preferably, for the light source a laser source is selected which is operable with pulse durations in the range of 1 - 200 ns. More preferably, the operating wavelength of the light source is tunable during use.

By using a suitably designed optical fiber based light delivery brought preferably in the proximity of the lesion the entire or a large part of the lesion may be illuminated so that the photoacoustic image for a corresponding large volume may be visualized.

In an embodiment of the system according to the invention it further comprises an ultrasonic unit adapted to enable an image-guided insertion of the biopsy needle into the target region and to detect photoacoustic signal generated by the target region.

It is found to be particularly advantageous to use the same ultrasonic unit for guiding the insertion of the biopsy needle provided with the optical fiber and to acquire the images generated of the tissue in response to the light irradiation.

In particular, substantially real-time visualisation of the resulting photoacoustic image using the same ultrasonic probe as used for the CNB methods may improve the biopsy quality as the sample may be accurately extracted from the desired pre-identified portion of the target region.

In a further embodiment of the system according to the invention the optical fiber is provided with beam shaping optics which is preferably integrated into the fiber. For example, the optical fiber may be provided with beam-diffusing optics, or, alternatively, the optical fiber may be provided with beam directing optics, or, alternatively beam sizing optics.

It is found advantageous to provide suitable light shaping or light conducting elements in the optical fiber for further improving spatial sensitivity of the system according to the invention. It is also possible that the light source is dynamically tuned, so that the wavelength is changed continuously or step-wise for illuminating the same portion of the tissue with different wavelengths generating different photoacoustic images. This may further increase the sensitivity of the system according to the invention.

In a still further embodiment of the system according to the invention the biopsy needle is provided with a plurality of optical fibers adapted to conduct light from the source towards the target region.

It is found that with this particular embodiment the accuracy of the system is still further increased. More in particular, it is possible that each or some of the plurality of the optical fibers conduct different light signals to substantially the same or neighbouring spots. With this feature sensitivity and specificity may further be increased.

In a still further embodiment of the system according to the invention the needle body is at least partially manufactured from a light guiding material. For example, a wall of the needle body may be manufactured from the light guiding material. Alternatively, the needle body may be fully manufactured form the light guiding material.

It is found to be particularly advantageous to implement at least a portion of the needle as an optical fiber. In this case the complexity of the needle may still further be decreased.

In a still further embodiment of the system according to the invention it comprises a plurality of biopsy needles adapted to conduct light from a remote light source via the distal portion towards the target region.

A method of generating photoacoustic images according to the invention comprises the steps of:
- generating photoacoustic signals using a pulsed or modulated light source having a wavelength in the range of 200 - 2500 nm;
- using a biopsy needle provided with an optical fiber to illuminate a target region using said light; optionally
- using the ultrasound unit to enable image guided needle insertion for detecting the photoacoustic signals.

It will be appreciated that for implementing the method the biopsy needle comprising the optical fiber may be pre-inserted. The method according to the invention is advantageous with respect to the prior art because the light inducing an acoustic response is deposited inside or at a surface of the tissue of interest.

A biopsy needle according to the invention comprises a needle body with a distal portion, the needle body being provided with an optical fiber adapted to conduct light from a remote light source via the distal portion towards a target region, wherein the optical fiber is provided with integrated beam shaping optics. Preferably, the optical fiber is provided with beam-diffusing optics or beam directing optics.

In an alternative embodiment, the needle according may be provided with a plurality of optical fibers adapted to conduct light from the source towards the target region.

In a still further embodiment of the needle according to the invention the needle body is at least partially manufactured from a light guiding material. This feature is found to be advantageous because the light intensity may be increased on one hand, and the complexity of the biopsy needle may be reduced on the other hand.

These and other aspects of the invention will be discussed in more detail with reference to drawings, wherein like reference signs represent like elements. It will be appreciated that the drawings are presented for illustrative purposes and may not be used for limiting the scope of the appended claims.

### BRIEF DESCRIPTION OF THE DRAWINGS

Figure 1 presents in a schematic way an embodiment of the system according to an aspect of the invention.
Figure 2 presents in a schematic way an embodiment of a biopsy needle according to an aspect of the invention.
Figure 3 presents in a schematic way a further embodiment of a biopsy needle according to a further aspect of the invention.
Figure 4 presents in a schematic way a still further embodiment of a biopsy needle according to a still further aspect of the invention.

### DETAILED DESCRIPTION OF THE DRAWINGS

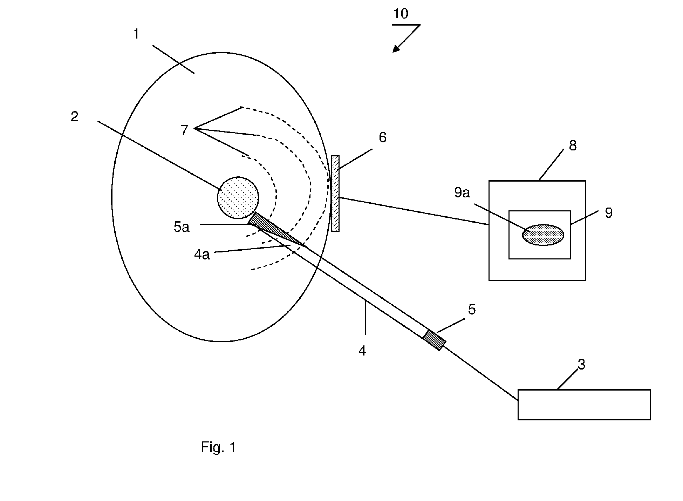

Figure 1 presents in a schematic way an embodiment of the system according to an aspect of the invention. The system 10 comprises a light source 3 arranged to generate pulsed or modulated light having a wavelength in the range of 200 - 2500 nm. The system 10 further comprises a biopsy needle having a needle body 4 with a distal portion 4a, the needle body being provided with an optical fiber 5 having a distal portion 5a and adapted to conduct the light from the light source 3 via the distal portion 5a towards a target region 2 located inside a patient 1.

Photoacoustic signals 7 generated in the target region 2 are advantageously intercepted by a suitable detector, such as a per se known ultrasonic probe 6. The ultrasonic probe 6 may be connected to a data processing system 8 for visualizing suitable images 9a on a display 9. In accordance with an aspect of the invention an improved photoacoustic system is provided wherein conducting of tissue biopsy is substantially improved due to provision of a biopsy needle provided with an integrated optical fibre.

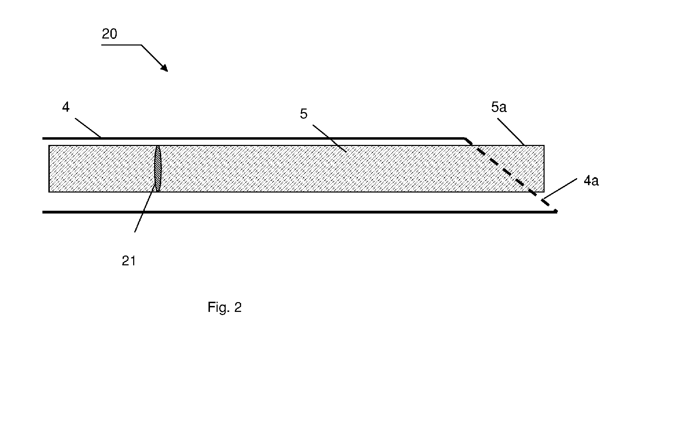

Figure 2 presents in a schematic way an embodiment of a biopsy needle according to an aspect of the invention. The biopsy needle 4 in accordance with an aspect of the invention comprises a distal portion 4a conceived to puncture tissue and to aspirate suitable portion of a target region. Inside the biopsy needle according to an aspect of the invention an optical fiber 5 having a distal portion 5a is provided. It will be appreciated that the respective dimensions of the biopsy needle 4 and the optical fiber 5 are exaggerated for clarity purposes. It will be further appreciated that the distal portion of the optical fiber 5a may protrude further than the distal portion 4a of the biopsy needle for increasing a spatial angle for the light emanating from the distal portion 5a. Generally, the distal portion 5a may be about 1 ― 3 mm protruding from the distal portion from of the biopsy needle. However, it is also possible that the optical cable does not protrude from the distal portion 4a of the biopsy needle 4. Still in particular, it is possible that the distal portion of the optical fiber 5a has substantially the same angulation (not shown) as the angle of the distal portion 4a of the optical fiber. In this case the optical fiber may be arranged to be substantially flush with the en surface of the distal portion 4a.

In accordance with a further aspect of the invention the optical fiber may be provided with suitable beam shaping optics 21. Various beam shaping elements, including but not limiting to a lens, a prism and so forth are contemplated. Alternatively, the optical element 21 may be arranged for beam-diffusing. Still alternatively, the optical element 21 may be arranged for directing the light in the fiber 5. Still alternatively, the optical element may be arranged for enabling suitable beam sizing. However, it will be appreciated that the beam shaping optics 21 can be situated anywhere along the optical delivery path including at the distal part of the needle. Further, the beam shaping optics is not necessarily arranged at one position, but may be distributed along the optical delivery.

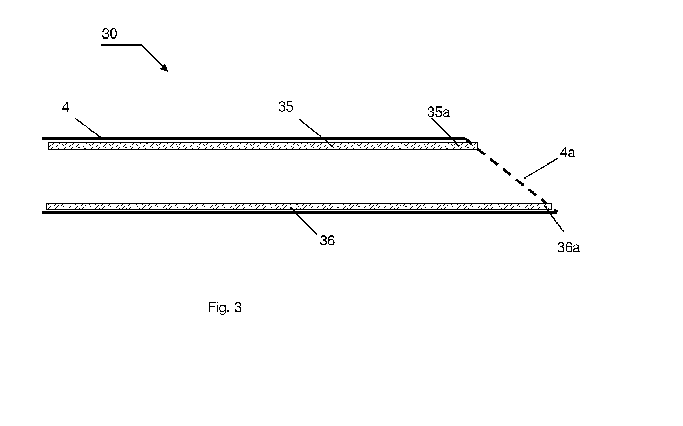

Figure 3 presents in a schematic way a further embodiment 30 of a biopsy needle according to a further aspect of the invention. In accordance with the present embodiment, the biopsy needle 4 is provided with a plurality of optical fibers 35, 36 (two being shown), which may be arranged along the walls of the needle 4. In this way the inner volume of the needle 4 may be optimally used for tissue aspiration. In accordance with the aspects of the invention discussed with reference to Figure 2, the optical fibers 35, 36 may have respective distal portions 35a, 36a which may be protruding from the distal portion 4a of the needle 4. Likewise, the distal portions 35a, 36a may be shaped to have the same angulation as the angulation of the distal portion 4a of the needle. Next to this, the optical fibers 35, 36 may be provided with a suitable optical element as is discussed with reference to Figure 2. It is found that when some of the plurality of the optical fibers 35, 36 conduct different light signals to substantially the same or neighbouring spots on the target region sensitivity and specificity may further be increased.

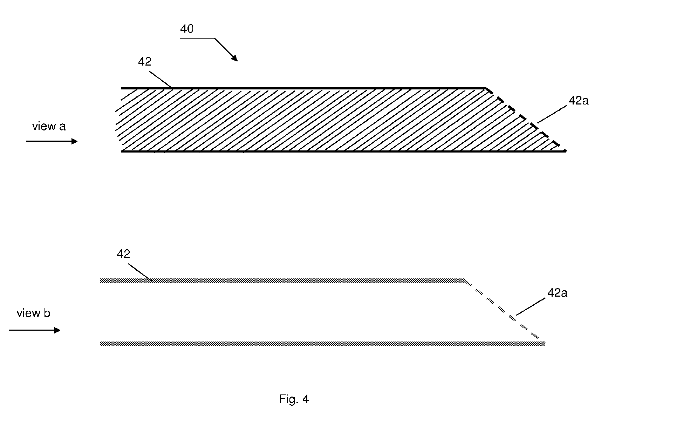

Figure 4 presents in a schematic way a still further embodiment 40 of a biopsy needle according to a still further aspect of the invention. In view "a" the body of the biopsy needle 42 is manufactured from a light guiding material. This feature simplifies the overall construction of the biopsy needle. In addition, by increasing the volume of the light conducting material a higher response by means of photoacoustic signal may be expected. This may lead to an improved quality of the photoacoustic images.

In view "b" an alternative embodiment is shown wherein the walls of the biopsy needle are manufactured or are covered by a light conducting material. For example, a suitable layer of a light conducting material may be provided on an outer or an inner surface of the needle wall. This embodiment is less costly than the embodiment shown in view "a" still having an improved signal response from the tissue.

It will be appreciated that although specific embodiments of the system and the biopsy needle according to the invention are discussed separately for clarity purposes, interchangeability of compatible features discussed with reference to isolated figures is envisaged. While specific embodiments have been described above, it will be appreciated that the invention may be practiced otherwise than is described. The descriptions above are intended to be illustrative, not limiting. Thus, it will be apparent to one skilled in the art that modifications may be made to the invention as described in the foregoing without departing from the scope of the claims set out below.

## Claims

1. A system for enabling generation of photoacoustic images comprising a light source, a biopsy needle having a needle body with a distal portion, the needle body being provided with an optical fiber adapted to conduct light from the light source via the distal portion towards a target region, wherein the light source comprises a pulsed or modulated light source having a wavelength in the range of 200 - 2500 nm.

2. The system according to claim 1, wherein the light source is a laser source operating with 1 - 200 ns pulses.

3. The system according to claim 1 or 2, further comprising an ultrasonic unit adapted to enable an image-guided insertion of the biopsy needle into the target region and to detect photoacoustic signal generated by the target region.

4. The system according to any one of the preceding claims, wherein the optical fiber is provided with beam shaping optics.

5. The system according to any one of the preceding claims, wherein the optical fiber is provided with beam-diffusing optics.

6. The system according to any one of the preceding claims 1 ― 5, wherein the optical fiber is provided with beam directing optics.

7. The system according to any one of the preceding claims 1 ― 6, wherein the optical fiber is provided with beam sizing optics.

8. The system according to any one of the preceding claims, wherein the biopsy needle is provided with a plurality of optical fibers adapted to conduct light from the source towards the target region.

9. The system according to any one of the preceding claims, wherein the needle body is at least partially manufactured from a light guiding material.

10. The system according to claim 8, wherein the needle body is fully manufactured form the light guiding material.

11. The system according to claim 8 or 9, wherein a wall of the needle body is manufactured from the light guiding material.

12. The system according to any one of the preceding claims, comprising a plurality of biopsy needles adapted to conduct light from a remote light source via the distal portion towards the target region.

13. The system according to any one of the preceding claims, wherein the operating wavelength of the light source is tunable.

14. A method of generating photoacoustic images comprises the steps of:
- generating photoacoustic signals using a pulsed or modulated light source having a wavelength in the range of 200 - 2500 nm;
- using a biopsy needle provided with an optical fiber to illuminate a target region using said light.

15. A biopsy needle having a needle body with a distal portion, the needle body being provided with an optical fiber adapted to conduct light from a remote light source via the distal portion towards a target region, wherein the optical fiber is provided with beam shaping optics.

16. The needle according to claim 15, wherein the optical fiber is provided with beam-diffusing optics.

17. The needle according to claim 15 or 16, wherein the optical fiber is provided with beam directing optics.

18. The needle according to claim 15, wherein the beam shaping optics is adapted for enabling beam sizing.

19. The needle according to any one of the preceding claims 15 - 18, wherein the biopsy needle is provided with a plurality of optical fibers adapted to conduct light from the source towards the target region.

20. The needle according to any one of the preceding claims 15 - 19, wherein the needle body is at least partially manufactured from a light guiding material.
